(19) 

**Europäisches Patentamt**
**European Patent Office**
**Office européen des brevets**

(11) **EP 3 873 974 B1**

(12) **EUROPEAN PATENT SPECIFICATION**

(45) Date of publication and mention
of the grant of the patent:
**06.05.2026  Bulletin 2026/19**

(21) Application number: **19794398.8**

(22) Date of filing: **23.10.2019**

(51) International Patent Classification (IPC):
**A61L 15/42** *(2006.01)*  **C08J 3/24** *(2006.01)*
**C08J 3/12** *(2006.01)*  **C08J 3/075** *(2006.01)*
**A61L 15/60** *(2006.01)*

(52) Cooperative Patent Classification (CPC):
(C-Sets available)
**C08J 3/075; A61L 15/42; A61L 15/60; C08J 3/12;**
**C08J 3/245;** C08J 2333/02            (Cont.)

(86) International application number:
**PCT/EP2019/078877**

(87) International publication number:
**WO 2020/089013 (07.05.2020 Gazette 2020/19)**

(54) **PROCESS FOR PRODUCING LONG-TERM COLOR STABLE SUPERABSORBENT POLYMER PARTICLES**

VERFAHREN ZUR HERSTELLUNG VON LANGZEITFARBSTABILEN SUPERABSORBIERENDEN POLYMERTEILCHEN

PROCÉDÉ DE PRODUCTION DE PARTICULES POLYMÈRES SUPERABSORBANTES STABLES EN COULEUR DE LONGUE DURÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.10.2018  PCT/CN2018/112364**

(43) Date of publication of application:
**08.09.2021  Bulletin 2021/36**

(73) Proprietor: **BASF SE**
**67056 Ludwigshafen am Rhein (DE)**

(72) Inventors:
• **BAUMANN, Katrin**
**67056 Ludwigshafen (DE)**
• **BAUER, Stephan**
**67056 Ludwigshafen (DE)**
• **DANIEL, Thomas**
**67056 Ludwigshafen (DE)**

• **HERFERT, Norbert**
**Shanghai 200137 (CN)**
• **BAUDUIN, Christophe**
**67056 Ludwigshafen (DE)**

(74) Representative: **BASF IP Association**
**BASF SE**
**GBI - Z078**
**67056 Ludwigshafen (DE)**

(56) References cited:
**EP-A2- 0 248 437           WO-A1-2016/134905**
**CN-A- 101 045 789       US-A1- 2007 078 231**
**US-A1- 2010 298 513**

• **DATABASE WPI Week 201230, Derwent World Patents Index; AN 2012-B49183, XP002796869**
• **DATABASE WPI Week 201582, Derwent World Patents Index; AN 2015-51932Q, XP002796870**

(52) Cooperative Patent Classification (CPC): (Cont.)

C-Sets
**A61L 15/60, C08L 33/02**

**Description**

The invention relates to a process for producing long-term color stable superabsorbent polymer particles, comprising polymerization of a monomer solution, drying the resulting polymer gel, optionally grinding and classifying the resulting dried polymer gel and thermally surface post-crosslinking and cooling the resulting polymer particles, wherein a thermal surface post-crosslinker and hydrogen peroxide are added to the polymer particles prior to the thermal surface post-crosslinking.

Superabsorbent polymer particles are used to produce diapers, tampons, sanitary napkins and other hygiene articles, but also as water-retaining agents in market gardening. The superab-sorbent polymer particles are often also referred to as "absorbent resins", "superabsorbents", "superabsorbent polymers", "absorbent polymers", "absorbent gelling materials", "hydrophilic polymers" or "hydrogels".

The production of superabsorbent polymer particles is described in the monograph "Modern Superabsorbent Polymer Technology", F.L. Buchholz and A.T. Graham, Wiley-VCH, 1998, pages 71 to 103.

The properties of superabsorbent polymer particles can be adjusted, for example, via the amount of crosslinker used. With increasing amount of crosslinker, the centrifuge retention capacity (CRC) falls and the absorption under a pressure of 21.0 $g/cm^2$ (AUL) passes through a maximum.

To improve the application properties, for example permeability of the swollen gel bed (SFC) in the diaper and absorption under a pressure of 49.2 $g/cm^2$ (AUHL), superabsorbent polymer particles are generally surface post-crosslinked. This increases the degree of crosslinking of the particle surface, which allows the absorption under a pressure of 49.2 $g/cm^2$ (AUHL) and the centrifuge retention capacity (CRC) to be at least partly de-coupled. This surface post-crosslinking can be performed in the aqueous gel phase. Preferably, however, dried, ground and screened-off polymer particles (base polymer) are surface coated with a surface post-crosslinker, thermally surface post-crosslinked and dried. Crosslinkers suitable for this purpose are compounds which can form covalent bonds with at least two carboxylate groups of the superabsorbing polymer particles.

A problem which often occurs in superabsorbent polymer particles is that of discoloration, which occurs in the course of storage at elevated temperature or elevated air humidity. Such conditions often occur in the course of storage in tropical or subtropical countries. Under such conditions, superabsorbent polymer particles tend to yellow; they may even take on a brown or even almost black color. This discoloration of the actually colorless superabsorbent polymer particles is unsightly and undesired, since it is visible especially in the desired thin hygiene products, and consumers reject unsightly hygiene products. The cause of the discoloration has not been entirely clarified, but reactive compounds such as residual monomers from the polymerization, the use of some initiators, impurities in the monomers or in the neutralizing agent, surface post-crosslinkers or stabilizers in the monomers used appear to play a role.

EP 1 770 113 A1 discloses surface post-crosslinking in the presence of peroxides for reduction of residual monomers.

WO 2006/062258 A2 discloses a process for surface post-crosslinking by adding a watersoluble polymerization initiator and irradiating the mixture with active energy rays. The process does not require any heating.

WO 2010/096595 A2 discloses superabsorbent polymers having antimicrobial properties.

The superabsorbent polymers are coated with 0.5 to 20% by weight of hydrogen peroxide and dried.

EP 2 915 548 A1 discloses a process for production of superabsorbent particles including the addition of peroxides after polymerization.

CN 101 045 789 A, CN 102 311 599 A, WO 2016/134905 A1, CN 104 744 711 A, US 2007/078231 A1, EP 0 248 437 A2 and US 2010/298513 A1 disclose a process for production of superabsorbent particles.

It was an object of the present invention to provide a process for producing superabsorbent polymer particles having an improved long-term color stability.

The object was achieved by a process for producing long-term color stable superabsorbent polymer particles, comprising polymerization of a monomer solution, comprising

  a) partly neutralized acrylic acid,
  b) 0.05 to 1.5% by weight, based on acrylic acid prior to neutralization, of at least one crosslinker, and
  c) at least one initiator, drying the resulting polymer gel, optionally grinding and classifying the resulting dried polymer gel and thermally surface post-crosslinking and cooling the resulting polymer particles, wherein 0.005 to 5% by weight of a thermal surface post-crosslinker and 0.016 to 0.080% by weight of hydrogen peroxide, based in each case on the polymer particles, are added to the polymer particles prior to the thermal surface post-crosslinking.

The thermal surface post-crosslinker is added to the polymer particles prior to the thermal surface post-crosslinking in an amount of preferably from 0.010 to 2% by weight, more preferably from 0.015 to 1% by weight, based on the polymer particles.

Suitable surface post-crosslinker are, for example, polyfunctional amines, polyfunctional amido amines, polyfunctional epoxides, di- or polyfunctional alcohols, $\beta$ - hydroxyalkylamides, cyclic carbonates, 2-oxazolidinone and derivatives thereof, bis- and poly-2-oxazolidinones, 2-oxotetra-hydro-1,3-oxazine and derivatives thereof, cyclic ureas, bicyclic amide acetals, oxetanes, cyclic ureas and morpholine-2,3-dione and derivatives thereof.

Hydrogen peroxide is added to the polymer particles prior to the thermal surface post-crosslinking in an amount of preferably from 0.018 to 0.065% by weight, most preferably from 0.020 to 0.050% by weight, based on the polymer particles.

The temperature of the polymer particles during thermal surface post-crosslinking is preferably from 110 to 220° C, more preferably from 120 to 200° C and most preferably from 130 to 190° C. Lower temperatures may be possible during heating-up of the polymer particles.

The residence time of the polymer particles in the thermal surface post-crosslinking is preferably from 10 to 120 minutes, more preferably from 15 to 90 minutes, most preferably from 20 to 60 minutes. The residence time is the residence time of the polymer particles in the apparatus that is used for thermal surface post-crosslinking.

In a preferred embodiment of the present invention, the monomer solution comprise a polymerization inhibitor in an amount of preferably from 0.001 to 0.020 % by weight, more preferably from 0.002 to 0.015% by weight, most preferably from 0.004 to 0.010% by weight, based on acrylic acid prior to neutralization.

Suitable polymerization inhibitors are, for example, hydroquinone monomethyl ether (MEHQ) and alpha-tocopherol (vitamin E). Hydroquinone monomethyl ether (MEHQ) is preferred.

In a preferred embodiment of the present invention, a chelating agent is added to the polymer particles during or after the cooling in an amount of preferably from 0.005 to 1.0 % by weight, more preferably from 0.010 to 0.7% by weight, most preferably from 0.015 to 0.5% by weight, based on the polymer particles.

Suitable chelating agents are, for example, citric acid, tartaric acid, iminodiacetic acid, hydroxyethyliminodiacetic acid, nitrilotriacetic acid, nitrilotripropionic acid, ethylenediamine-etetraacetic acid, diethylenetriaminepentaacetic acid, triethylenetetraaminehexaacetic acid, N,N-bis(2-hydroxyethyl)glycine, trans-1,2-diaminocyclohexanetetraacetic acid and 1-hydroxy-ethane-1,1-diphosphonic acid, and salts thereof. 1-hydroxyethane-1,1-diphosphonic acid and/or salts thereof are preferred.

The surface post-crosslinking is performed by heating. There is no need for any irradiation with active energy rays.

The invention is based on the finding that the long-term color stability of superabsorbent polymers can be increased by treatment with hydrogen peroxide prior to thermal post-crosslinking. Too high amounts of hydrogen peroxides have a negative impact on the absorption under high load (AUHL).

Addition of hydrogen peroxide after thermal post-crosslinking or using other peroxides is less effective.

The present invention further provides superabsorbent polymer particles obtainable by the process according to the invention.

The superabsorbent polymer particles produced by the process according to the invention have a centrifuge retention capacity (CRC) of at least 40 g/g, preferably at least 41 g/g, more preferably at least 42 g/g. The centrifuge retention capacity (CRC) of the superabsorbent polymer particles is typically less than 60 g/g.

The superabsorbent polymer particles produced by the process according to the invention have an absorption under high load (AUHL) of at least 20 g/g, preferably at least 22 g/g, more preferably at least 24 g/g. The absorption under high load (AUHL) of the superabsorbent polymer particles is typically less than 50 g/g.

The superabsorbent polymer particles produced by the process according to the invention have a yellowness index (YI) of YI D1925 (2/C) after 14 days of aging at 70° C and 80% relative humidity of not more than 54, preferably not more than 52, more preferably not more than 50.

The production of the superabsorbents is described in detail hereinafter:
The superabsorbents are produced by polymerizing a monomer solution, and are typically water-insoluble.

Suitable crosslinkers b) are compounds having at least two groups suitable for crosslinking.

Such groups are, for example, ethylenically unsaturated groups which can be polymerized free-radically into the polymer chain, and functional groups which can form covalent bonds with the acid groups of acrylic acid. In addition, polyvalent metal salts which can form coordinate bonds with at least two acid groups of acrylic acid are also suitable as crosslinkers b).

Crosslinkers b) are preferably compounds having at least two polymerizable groups which can be polymerized free-radically into the polymer network. Suitable crosslinkers b) are, for example, ethylene glycol dimethacrylate, diethylene glycol diacrylate, polyethylene glycol diacrylate, allyl methacrylate, trimethylolpropane triacrylate, triallylamine, tetraallylammonium chloride, tetraallyloxyethane, as described in EP 0 530 438 A1, di- and triacrylates, as described in EP 0 547 847 A1, EP 0 559 476 A1, EP 0 632 068 A1, WO 93/21237 A1, WO 2003/104299 A1, WO 2003/104300 A1, WO 2003/104301 A1 and DE 103 31 450 A1, mixed acrylates which, as well as acrylate groups, comprise further ethylenically unsaturated groups, as described in DE 103 31 456 A1 and DE 103 55 401 A1, or crosslinker mixtures, as described, for example, in DE 195 43 368 A1, DE 196 46 484 A1, WO 90/15830 A1 and WO 2002/032962 A2.

Preferred crosslinkers b) are pentaerythrityl triallyl ether, tetraallyloxyethane, methylenebismeth-acrylamide, 15-tuply ethoxylated trimethylolpropane triacrylate, polyethylene glycol diacrylate, trimethylolpropane triacrylate and triallylamine.

Very particularly preferred crosslinkers b) are the polyethoxylated and/or -propoxylated glycerols which have been esterified with acrylic acid or methacrylic acid to give di- or triacrylates, as described, for example, in WO 2003/104301 A1. Di- and/or triacrylates of 3-to 10-tuply ethoxylated glycerol are particularly advantageous. Very particular preference is given to di- or triacrylates of 1- to 5-tuply ethoxylated and/or propoxylated glycerol. Most preferred are the triacrylates of 3- to 5-tuply ethoxylated and/or propoxylated glycerol, especially the triacrylate of 3-tuply ethoxylated glycerol.

The amount of crosslinker b) is 0.05 to 1.5% by weight, preferably 0.1 to 1% by weight and more preferably 0.3 to 0.6% by weight, based in each case on acrylic acid prior to neutralization. With rising crosslinker content, the centrifuge retention capacity (CRC) falls and the absorption under a pressure of 21.0 $g/cm^2$ passes through a maximum.

The initiators c) used may be all compounds which generate free radicals under the polymerization conditions, for example thermal initiators, redox initiators, photoinitiators. Suitable redox initiators are sodium peroxodisulfate/ascorbic acid, hydrogen peroxide/ascorbic acid, sodium peroxodisulfate/sodium bisulfite and hydrogen peroxide/sodium bisulfite. Preference is given to using mixtures of thermal initiators and redox initiators, such as sodium peroxodisulfate/hydrogen peroxide/ascorbic acid. However, the reducing component used is preferably disodium 2-hydroxy-2-sulfonatoacetate or a mixture of disodium 2-hydroxy-2-sulfin-atoacetate, disodium 2-hydroxy-2-sulfonatoacetate and sodium bisulfite. Such mixtures are obtainable as Brüggolite® FF6 and Brüggolite® FF7 (Brüggemann Chemicals; Heilbronn; Germany).

Typically, an aqueous monomer solution is used. The water content of the monomer solution is preferably from 40 to 75% by weight, more preferably from 45 to 70% by weight and most preferably from 50 to 65% by weight. It is also possible to use monomer suspensions, i.e. monomer solutions with excess sodium acrylate. With rising water content, the energy requirement in the subsequent drying rises, and, with falling water content, the heat of polymerization can only be removed inadequately.

For optimal action, the preferred polymerization inhibitors require dissolved oxygen. The monomer solution can therefore be freed of dissolved oxygen before the polymerization by inertization, i.e. flowing an inert gas through, preferably nitrogen or carbon dioxide. The oxygen content of the monomer solution is preferably lowered before the polymerization to less than 1 ppm by weight, more preferably to less than 0.5 ppm by weight, most preferably to less than 0.1 ppm by weight.

For better control of the polymerization reaction, it is optionally possible to add all known chelating agents to the monomer solution or suspension or to the raw materials thereof. Suitable chelating agents are, for example, phosphoric acid, diphosphoric acid, triphosphoric acid, polyphosphoric acid, citric acid, tartaric acid, or salts thereof.

Further suitable examples are iminodiacetic acid, hydroxyethyliminodiacetic acid, nitrilotriacetic acid, nitrilotripropionic acid, ethylenediaminetetraacetic acid, diethylenetriaminepentaacetic acid, triethylenetetraaminehexaacetic acid, N,N-bis(2-hydroxyethyl)glycine and trans-1,2-dia-minocyclohexanetetraacetic acid, and salts thereof. The amount used is typically 1 to 30 000 ppm based on the monomers a), preferably 10 to 1000 ppm, preferentially 20 to 600 ppm, more preferably 50 to 400 ppm, most preferably 100 to 300 ppm.

The monomer solution is polymerized. Suitable reactors are, for example, kneading reactors or belt reactors. In the kneader, the polymer gel formed in the polymerization of an aqueous monomer solution or suspension is comminuted continuously by, for example, contrarotatory stirrer shafts, as described in WO 2001/038402 A1. Polymerization on the belt is described, for example, in DE 38 25 366 A1 and US 6,241,928. Polymerization in a belt reactor forms a polymer gel which has to be comminuted in a further process step, for example in an extruder or kneader.

To improve the drying properties, the comminuted polymer gel obtained by means of a kneader can additionally be extruded.

The acid groups of the resulting polymer gels have typically been partially neutralized. Neutralization is preferably carried out at the monomer stage. This is typically accomplished by mixing in the neutralizing agent as an aqueous solution or preferably also as a solid. The degree of neutralization is preferably from 50 to 85 mol%, more preferably from 60 to 80 mol% and most preferably from 65 to 75 mol%, for which the customary neutralizing agents can be used, preferably alkali metal hydroxides, alkali metal oxides, alkali metal carbonates or alkali metal hydrogencarbonates and also mixtures thereof. Instead of alkali metal salts, it is also possible to use ammonium salts. Particularly preferred alkali metals are sodium and potassium, but very particular preference is given to sodium hydroxide, potassium hydroxide and also mixtures thereof.

The resulting polymer gel is dried. The driers are not subject to any restriction. However, the drying of the polymer gel is preferably performed with a belt drier until the residual moisture content is preferably 0.5 to 10% by weight, more preferably 1 to 7% by weight and most preferably 2 to 5% by weight, the residual moisture content being determined by EDANA recommended test method No. WSP 230.2 (05) "Mass Loss Upon Heating". In the case of to high a residual moisture content, the dried polymer gel has too low a glass transition temperature $T_g$ and can be processed further only with difficulty. In the case of too low a residual moisture content, the dried polymer gel is too brittle and, in the subsequent grinding steps, undesirably large amounts of polymer particles with an excessively low particle size are obtained ("fines").

The solids content of the gel before the drying is preferably from 25 to 90% by weight, more preferably from 35 to 70% by weight and most preferably from 40 to 60% by weight. However, a fluidized bed drier or a paddle drier may optionally also be used for drying purposes.

Subsequently, the dried polymer gel is can be ground and classified. The apparatus used for grinding may typically be single- or multistage roll mills, preferably two- or three-stage roll mills, pin mills, hammer mills or vibratory mills.

The mean particle size of the polymer particles removed as the product fraction is preferably at least 200 $\mu$m, more preferably from 250 to 600 $\mu$m and very particularly from 300 to 500 $\mu$m. The mean particle size of the product fraction may be determined by means of EDANA recommended test method No. WSP 220.2 (05) "Particle Size Distribution", where the proportions by mass of the screen fractions are plotted in cumulated form and the mean particle size is determined graphically. The mean particle size here is the value of the mesh size which gives rise to a cumulative 50% by weight.

The proportion of particles with a particle size of at least 150 $\mu$m is preferably at least 90% by weight, more preferably at least 95% by weight, most preferably at least 98% by weight.

Polymer particles with too small a particle size lower the saline flow conductivity (SFC). The proportion of excessively small polymer particles ("fines") should therefore be low.

Excessively small polymer particles are therefore typically removed and recycled into the process. This is preferably done before, during or immediately after the polymerization, i.e. before the drying of the polymer gel. The excessively small polymer particles can be moistened with water and/or aqueous surfactant before or during the recycling.

It is also possible to remove excessively small polymer particles in later process steps, for example after the surface post-crosslinking or another coating step. In this case, the excessively small polymer particles recycled are surface post-crosslinked or coated in another way, for example with fumed silica or precipitated silica.

When a kneading reactor is used for polymerization, the excessively small polymer particles are preferably added during the last third of the polymerization.

When the excessively small polymer particles are added at a very early stage, for example actually to the monomer solution, this lowers the centrifuge retention capacity (CRC) of the resulting superabsorbents. However, this can be compensated for, for example, by adjusting the amount of crosslinker b) used.

When the excessively small polymer particles are added at a very late stage, for example not until an apparatus connected downstream of the polymerization reactor, for example an extruder, the excessively small polymer particles can be incorporated into the resulting polymer gel only with difficulty. Insufficiently incorporated, excessively small polymer particles are, however, detached again from the dried polymer gel during the grinding, are therefore removed again in the course of classification and increase the amount of excessively small polymer particles to be recycled.

The proportion of particles having a particle size of at most 850 $\mu$m is preferably at least 90% by weight, more preferably at least 95% by weight, most preferably at least 98% by weight.

The proportion of particles having a particle size of at most 600 $\mu$m is preferably at least 90% by weight, more preferably at least 95% by weight, most preferably at least 98% by weight.

Polymer particles of excessively large particle size lower the free swell rate (FSR). The proportion of excessively large polymer particles should therefore likewise be small.

Excessively large polymer particles are therefore typically removed and recycled into the grinding of the dried polymer gel.

However, it is also possible to dropletize an aqueous monomer solution and to polymerize the droplets obtained in a heated carrier gas stream. It is possible here to combine the process steps of polymerization and drying, as described in WO 2008/052971 A1 and WO 2011/026876 A1.

For this purpose, the monomer solution is metered into the reaction chamber by means of at least one hole to form droplets. The holes may, for example, be in a dropletizer plate.

A dropletizer plate is a plate with at least one hole, the liquid passing through the hole from the top. The dropletizer plate or the liquid can be oscillated, which generates a chain of ideally monodisperse droplets at each hole on the underside of the dropletizer plate. In a preferred embodiment, the dropletizer plate is not agitated.

The number and size of the holes are selected according to the desired capacity and droplet size. The droplet diameter is typically 1.9 times the diameter of the hole. What is important here is that the liquid to be dropletized does not pass through the hole too rapidly and the pressure drop across the hole is not too great. Otherwise, the liquid is not dropletized, but rather the liquid jet is broken up (sprayed) owing to the high kinetic energy. The Reynolds number based on the throughput per hole and the hole diameter is preferably less than 2000, more preferably less than 1600, especially preferably less than 1400 and most preferably less than 1200.

The dropletizer plate has preferably at least 5, more preferably at least 25 and most preferably at least 50 holes, and preferably up to 750, more preferably up to 500 and most preferably up to 250 holes. The diameter of the holes is selected according to the desired droplet size.

The diameter of the holes is preferably from 50 to 500 $\mu$m, more preferably from 100 to 300 $\mu$m and most preferably from 150 to 250 $\mu$m.

The temperature of the monomer solution on passage through the holes is preferably from 5 to 80° C, more preferably from 10 to 70° C and most preferably from 30 to 60° C.

The distance between the holes is preferably 10 to 50 mm, more preferably 12 to 40 mm and most preferably 15 to 30 mm. Excessively small distances lead to formation of agglomerates.

A carrier gas flows through the polymerization reactor. This carrier gas can be conducted through the reaction chamber in cocurrent or in countercurrent to the free-falling droplets of the monomer solution, preferably in cocurrent, i.e. from the bottom upward. After one pass, the carrier gas is preferably recycled at least partly into the reaction chamber as cycle gas, preferably to an extent of at least 50% and more preferably to an extent of at least 75%. Typically, a portion of the carrier gas is discharged after each pass, preferably up to 10%, more preferably up to 3% and most preferably up to 1%.

The oxygen content of the carrier gas is preferably from 0.5 to 15% by volume, more preferably from 1 to 10% by volume and most preferably from 2 to 7% by weight.

As well as oxygen, the carrier gas preferably comprises nitrogen. The nitrogen content of the carrier gas is preferably at least 80% by volume, more preferably at least 90% by volume and most preferably at least 95% by volume. Further suitable carrier gases are carbon dioxide, argon, xenon, krypton, neon and helium. It is also possible to use gas mixtures. The carrier gas may also be laden with steam and/or acrylic acid vapors.

The gas velocity is preferably set such that the flow in the polymerization reactor is directed, for example no convection currents opposed to the general flow direction are present, and is typically 0.1 to 2.5 m/s, preferably 0.3 to 1.5 m/s, more preferably from 0.5 to 1.2 m/s, especially preferably 0.6 to 1.0 m/s and most preferably 0.7 to 0.9 m/s.

The carrier gas flowing through the reactor is appropriately preheated to the reaction temperature upstream of the reactor.

Advantageously, the gas inlet temperature is regulated such that the gas outlet temperature, i.e. the temperature with which the carrier gas leaves the reaction chamber, is typically from 90 to 150° C, preferably from 100 to 140° C, more preferably from 105 to 135° C, especially preferably from 110 to 130° C and most preferably from 115 to 125° C.

The reaction can be performed under elevated pressure or under reduced pressure; a reduced pressure of down to 100 mbar relative to ambient pressure is preferred.

The reaction offgas, i.e. the gas leaving the reaction chamber, can, for example, be cooled in a heat exchanger. This condenses water and unconverted monomer a). Thereafter, the reaction offgas can at least partly be reheated and recycled into the reactor as cycle gas. A portion of the reaction offgas can be discharged and replaced by fresh carrier gas, in which case water and unconverted monomers a) present in the reaction offgas can be removed and recycled.

Particular preference is given to an integrated heating system, which means that some of the waste heat in the cooling of the offgas is used to heat the cycle gas.

The reactors may be trace-heated. The trace heating is adjusted such that the wall temperature is at least 5° C above the internal reactor temperature, and condensation at the reactor walls is reliably prevented.

The reaction product is subsequently thermally aftertreated and optionally dried down to the desired water content.

To improve the properties, the polymer particles (base polymer) are subsequently be thermally surface post-crosslinked. Suitable surface post-crosslinkers are compounds which comprise groups which can form covalent bonds with at least two acid groups of the polymer particles. Suitable compounds are, for example, polyfunctional amines, polyfunctional amido amines, polyfunctional epoxides, as described in EP 0 083 022 A2, EP 0 543 303 A1 and EP 0 937 736 A2, di- or polyfunctional alcohols, as described in DE 33 14 019 A1, DE 35 23 617 A1 and EP 0 450 922 A2, or $\beta$-hydroxyalkylamides, as described in DE 102 04 938 A1 and US 6,239,230.

Additionally described as suitable surface post-crosslinkers are cyclic carbonates in DE 40 20 780 C1, 2-oxazolidinone and derivatives thereof, such as 2-hydroxyethyl-2-oxazolidinone, in DE 198 07 502 A1, bis- and poly-2-oxazolidinones in DE 198 07 992 C1, 2-oxotetra-hydro-1,3-oxazine and derivatives thereof in DE 198 54 573 A1, N-acyl-2-oxazolidinones in DE 198 54 574 A1, cyclic ureas in DE 102 04 937 A1, bicyclic amide acetals in DE 103 34 584 A1, oxetanes and cyclic ureas in EP 1 199 327 A2 and morpholine-2,3-dione and derivatives thereof in WO 2003/031482 A1.

Preferred surface post-crosslinkers are ethylene carbonate, ethylene glycol diglycidyl ether, reaction products of polyamides with epichlorohydrin and mixtures of propylene glycol and 1,4-butanediol.

Very particularly preferred surface post-crosslinkers are 2-hydroxyethyloxazolidin-2-one, oxazolidin-2-one and 1,3-propanediol.

In addition, it is also possible to use surface post-crosslinkers which comprise additional polymerizable ethylenically unsaturated groups, as described in DE 37 13 601 A1.

The amount of surface post-crosslinker is 0.005 to 5% by weight, preferably 0.01 to 2% by weight and more preferably 0.015 to 1% by weight, based in each case on the polymer particles.

In a preferred embodiment of the present invention, polyvalent cations are applied to the particle surface in addition to the surface post-crosslinkers before, during or after the surface post-crosslinking.

The polyvalent cations usable in the process according to the invention are, for example, divalent cations such as the cations of zinc, magnesium, calcium, iron and strontium, trivalent cations such as the cations of aluminum, iron, chromium, rare earths and manganese, tetravalent cations such as the cations of titanium and zirconium. Possible counterions are chloride, bromide, sulfate, hydrogensulfate, carbonate, hydrogencarbonate, nitrate, phosphate, hydrogenphosphate, dihydrogenphosphate and carboxylate, such as acetate and lactate. Aluminum sulfate and aluminum lactate are preferred. Apart from metal salts, it is also possible to use polyamines as polyvalent cations.

The amount of polyvalent cation used is, for example, 0.001 to 1.5% by weight, preferably 0.005 to 1% by weight and more preferably 0.02 to 0.8% by weight, based on the polymer particles.

The surface post-crosslinking is typically performed in such a way that a solution of the surface post-crosslinker is sprayed onto the dried polymer particles. After the spray application, the polymer particles coated with surface post-crosslinker are dried thermally, and the surface post-crosslinking reaction can take place either before or during the drying.

The spray application of a solution of the surface post-crosslinker is preferably performed in mixers with moving mixing tools, such as screw mixers, disk mixers and paddle mixers. Particular preference is given to horizontal mixers such as paddle mixers, very particular preference to vertical mixers. The distinction between horizontal mixers and vertical mixers is made by the position of the mixing shaft, i.e. horizontal mixers have a horizontally mounted mixing shaft and vertical mixers a vertically mounted mixing shaft. Suitable mixers are, for example, horizontal Pflugschar® plowshare mixers (Gebr. Lödige Maschinenbau GmbH; Paderborn; Germany), Vrieco-Nauta continuous mixers (Hosokawa Micron BV; Doetinchem; the Netherlands), Processall Mixmill mixers (Processall Incorporated; Cincinnati; USA) and Schugi Flexomix® (Hosokawa Micron BV; Doetinchem; the Netherlands). However, it is also possible to spray on the surface post-crosslinker solution in a fluidized bed.

The surface post-crosslinkers are typically used in the form of an aqueous solution. The penetration depth of the surface post-crosslinker into the polymer particles can be adjusted via the content of non-aqueous solvent and total amount of solvent.

When exclusively water is used as the solvent, a surfactant is advantageously added. This improves the wetting behavior and reduces the tendency to form lumps. However, preference is given to using solvent mixtures, for example isopropanol/water, 1,3-propanediol/water and propylene glycol/water, where the mixing ratio in terms of mass is preferably from 20:80 to 40:60.

The thermal surface post-crosslinking is preferably performed in contact driers, more preferably paddle driers, most preferably disk driers. Suitable driers are, for example, Hosokawa Bepex® Horizontal Paddle Dryer (Hosokawa Micron GmbH; Leingarten; Germany), Hosokawa Bepex® Disc Dryer (Hosokawa Micron GmbH; Leingarten; Germany) and Nara Paddle Dryer (NARA Machinery Europe; Frechen; Germany). Moreover, fluidized bed driers may also be used.

The thermal surface post-crosslinking can be effected in the mixer itself, by heating the jacket or blowing in warm air. Equally suitable is a downstream drier, for example a shelf drier, a rotary tube oven or a heatable screw. It is particularly advantageous to effect mixing and drying in a fluidized bed drier.

The surface post-crosslinking temperatures are in the range of preferably 110 to 220° C, more preferably 120 to 200° C and most preferably 130 to 190° C. The residence time of the polymer particles in the thermal surface post-crosslinking is preferably from 10 to 120 minutes, more preferably from 15 to 90 minutes, most preferably from 20 to 60 minutes.

Subsequently, the surface post-crosslinked polymer particles can be classified again, excessively small and/or excessively large polymer particles being removed and recycled into the process.

To further improve the properties, the surface post-crosslinked polymer particles can be coated or remoisturized.

The remoisturizing is preferably performed at 30 to 80° C, more preferably at 35 to 70° C, most preferably at 40 to 60° C. At excessively low temperatures, the superabsorbents tend to form lumps, and, at higher temperatures, water already evaporates to a noticeable degree. The amount of water used for remoisturizing is preferably from 1 to 15% by weight, more preferably from 2 to 10% by weight and most preferably from 3 to 5% by weight, based on the polymer particles. The remoisturizing increases the mechanical stability of the polymer particles and reduces their tendency to static charging.

Suitable coatings for improving the free swell rate and the saline flow conductivity (SFC) are, for example, inorganic inert substances, such as water-insoluble metal salts, organic polymers, cationic polymers and di- or polyvalent metal cations. Suitable coatings for dust binding are, for example, polyols. Suitable coatings for counteracting the undesired caking tendency of the polymer particles are, for example, fumed silica, such as Aerosil® 200, or precipitated silica, such as Sipernat® D17, and surfactants, such as Span® 20.

Methods:

The measurements should, unless stated otherwise, be carried out at an ambient temperature of 23 ± 2° C and a relative atmospheric humidity of 50 ± 10%. The superabsorbent polymers are mixed thoroughly before the measurement.

Residual monomers (RAA)

The residual monomers in superabsorbent polymer particles are determined by EDANA recommended test method No. WSP 210.2 (04) "Determination of the Amount of Residual Monomers in Superabsorbent Materials".

Particle Size Distribution

The particle size distribution of the superabsorbent polymer particles is determined by the EDANA recommended test method No. WSP 220.2 (05) "Determination of Polyacrylate Superabsorbent Powders and Particle Size Distribution - Sieve Fractionation".

Roundness

The roundness is determined with the PartAn® 3001 L Particle Analysator (Microtrac Europe GmbH; Meerbusch; Germany). The roundness is defined as

$$Roundness = \frac{4\pi A}{U^2}$$

where A is the cross-sectional area and U is the cross-sectional circumference of the polymer particles. The roundness is the volume-average roundness.

For the measurement, the superabsorbent polymer particles are introduced through a funnel and conveyed to the falling shaft with a metering channel. While the particles fall past a light wall, they are recorded selectively by a camera. The images recorded are evaluated by the software in accordance with the parameters selected.

Moisture Content (MC)

The moisture content of the superabsorbent polymer particles is determined by the EDANA recommended test method No. WSP 230.2 (05) "Moisture Content - Weight Loss Upon Heating".

Centrifuge Retention Capacity (CRC)

The centrifuge retention capacity of superabsorbent polymer particles is determined by the EDANA recommended test method No. WSP 241.2 (05) "Gravimetric Determination of Fluid Retention Capacity in Saline Solution After Centrifugation", wherein for higher values of the centrifuge retention capacity lager tea bags have to be used.

Absorbency Under Load (AUL)

The absorbency under load of the superabsorbent polymer particles is determined by the EDANA recommended test method No. WSP 242.2 (05) "Gravimetric Determination of Absorption Under Pressure".

Absorbency Under High Load (AUHL)

The absorbency under high load of the superabsorbent polymer particles is determined analogously to the EDANA recommended test method No. WSP 242.2 (05) "Gravimetric Determination of Absorption Under Pressure", except using a weight of 49.2 g/cm$^2$ instead of a weight of 21.0 g/cm$^2$.

Flow Rate

The flow rate of the superabsorbent polymer particles is determined by the EDANA recommended test method No. WSP 250.2 (05) "Gravimetric Determination of Flowrate".

Bulk Density

The bulk density of the superabsorbent polymer particles is determined by the EDANA recom-mended test method No. WSP 260.2 (05) "Gravimetric Determination of Density".

Extractables (Ext. 16h)

The content of extractable constituents in superabsorbent polymer particles is determined by the EDANA recommended test method No. WSP 270.2 (05) "Determination of Extractable Polymer Content by Potentiometric Titration".

Caking (40° C/80%r.h./1h)

5 g of the superabsorbent polymer particles are placed in an aluminum weighing dish (57 mm x 15 mm) and stored for 1 hour at 40° C and 80% relative humidity. The samples are cooled down to ambient temperature and weighed. After sieving over a sieve of 1.68 mm hole size (ASTM No. 12, Diameter of the sieve > 57 mm and < 100 mm), the amount which passes through the sieve is weighed to determine the mass of the non-caking polymer particles. The sieving process is described as follows:
Carefully take the aluminum dish containing hydrated polymer and hold upright in one hand. Invert the sieve-pan assembly over the dish and in one continuous motion, gently invert the sieve, pan and weighing dish-containing polymer, such that the dish is now inverted on top of the sieve screen. Add the lid to the sieve screen including the aluminum weighing dish and place the assembly in the sieve shaker. Vibrate the sieve assembly for one minute at 0.20 mm amplitude with a Retsch® Vibratory Sieve Shaker AS 200 control.
The percent of the particles which are non-caking is then determined by the following formula:

$$\text{Caked Polymer (\%)} = 100 - \left( \frac{W_{UNC} - W_{PAN}}{W_{HYD} - W_d} \right) x 100)$$

where $W_d$ is the weight of aluminum dish, $W_{HYD}$ is the weight of hydrated polymer plus aluminum dish before sifting, $W_{PAN}$ is the weight of the collection pan and $W_{UNC}$ is the weight of collection pan and hydrated polymer.

Color Value (CIE Color Numbers [L, a, b])

Measurement of the color value is done by means of a colorimeter model "LabScan XE Spectrometer" (HunterLab; Reston; U.S.A.) according to the CIELAB procedure (Hunterlab, Volume 8, 1996, Issue 7, pages 1 to 4). Colors are described by the coordinates L, a, and b of a three-dimensional system. L characterizes the brightness, whereby L = 0 is black and L = 100 is white. The values for a and b describe the position of the color on the color axis red/green resp. yellow/blue, whereby positive a values stand for red colors, negative a values for green colors, positive b values for yellow colors, and negative b values for blue colors.
The Yellowness Index (YI) of YI D1925 (2/C) is measured per ASTM D-1925, 2 deg./III. ° C. As higher the value as darker and yellower the color is.
The test was done using a Tissue Culture Dish (diameter of 35 mm and height of 10 mm) and a Port Plate Opening of 0.5 inch.
The measurement of the color value is in agreement with the tristimulus method according to DIN 5033-6.

Examples

Example 1 (base polymer)

The example was done analogously to Example 1 of WO 2016/134905 A1.
The process was performed in a concurrent spray drying plant with an integrated fluidized bed (27) as shown in figure 1 of WO 2016/134905 A1. The reaction zone (5) had a height of 22m and a diameter of 3.4m. The internal fluidized bed (IFB) had a diameter of 3m and a weir height of 0.25m.
The drying gas was fed via a gas distributor (3) at the top of the spray dryer. The drying gas was partly recycled (drying gas loop) via a cyclone as dust separation unit (9) and a condenser column (12). The drying gas was nitrogen that comprises from 1% to 4% by volume of residual oxygen. Prior to the start of polymerization the drying gas loop was filled with nitrogen until the residual oxygen was below 4% by volume. The gas velocity of the drying gas in the reaction zone (5) was 0.79m/s. The pressure inside the spray dryer was 4mbar below ambient pressure.
The temperature of the gas leaving the reaction zone (5) was measured at three points around the circumference at the end of the cylindrical part of the spray dryer as shown in figure 3 of WO 2016/134905 A1. Three single measurements (43) were used to calculate the average temperature (spray dryer outlet temperature). The drying gas loop was heated up and the dosage of monomer solution is started up. From this time the spray dryer outlet temperature was controlled to 114° C by adjusting the gas inlet temperature via the heat exchanger (20). The gas inlet temperature was 167° C.

The product accumulated in the internal fluidized bed (27) until the weir height was reached. Conditioned internal fluidized bed gas having a temperature of 105° was fed to the internal fluidized bed (27) via line (25). The gas velocity of the internal fluidized bed gas in the internal fluidized bed (27) was 0.65m/s. The residence time of the product was 150min. The temperature of the superabsorbent polymer particles in the internal fluidized bed (27) was 71° C.

The spray dryer offgas was filtered in cyclone as dust separation unit (9) and sent to a condenser column (12) for quenching/cooling. Excess water was pumped out of the condenser column (12) by controlling the (constant) filling level inside the condenser column (12). The water inside the condenser column (12) was cooled by a heat exchanger (13) and pumped counter-current to the gas. The water inside the condenser column (12) was set to an alkaline pH by dosing sodium hydroxide solution to wash out acrylic acid vapors.

The gas leaving the condenser column (12) was split to the drying gas inlet pipe (1) and the conditioned internal fluidized bed gas (25). The gas temperatures were controlled via heat exchangers (20) and (22). The hot drying gas was fed to the concurrent spray dryer via gas distributor (3). The gas distributor (3) consists of a set of plates providing a pressure drop of 2 to 4mbar depending on the drying gas amount.

The product was discharged from the internal fluidized bed (27) via rotary valve (28) into sieve (29). The sieve (29) was used for sieving off overs/lumps having a particle diameter of more than 800 $\mu$m.

The monomer solution was prepared by mixing first acrylic acid with 3-tuply ethoxylated glycerol triacrylate (internal crosslinker), secondly with 37.3% by weight sodium acrylate solution and thirdly with aqueous of disodium 1-hydroxyethane-1,1-diphosphonic acid (HDPA). The temperature of the resulting monomer solution was controlled to 10° C by using a heat exchanger and pumping in a loop. A filter unit having a mesh size of 250 $\mu$m was used in the loop after the pump. The initiators were metered into the monomer solution upstream of the dropletizer by means of static mixers (31) and (32) via lines (33) and (34) as shown in figure 1 of
WO 2016/134905 A1. sodium peroxodisulfate solution having a temperature of 20° C was added via line (33) and [2,2'-azobis[2-(2-imidazolin-2-yl)propane]dihydrochloride solution together was added via line (34). Each initiator was pumped in a loop and dosed via control valves to each dropletizer unit. A second filter unit having a mesh size of 140$\mu$m was used after the static mixer (32). For dosing the monomer solution into the top of the spray dryer three dropletizer units were used as shown in figure 4 of WO 2016/134905 A1.

A dropletizer unit consisted of an outer pipe (47) having an opening for the dropletizer cassette (49) as shown in figure 5 of WO 2016/134905 A1. The dropletizer cassette (49) was connected with an inner pipe (48). The inner pipe (48) having a PTFE block (50) at the end as sealing can be pushed in and out of the outer pipe (47) during operation of the process for maintenance purposes.

The dropletizer cassette (49) had 256 bores having a diameter of 170$\mu$m and a bore spacing of 15mm. The dropletizer cassette (49) consisted of a flow channel (56) having essential no stagnant volume for homogeneous distribution of the premixed monomer and initiator solutions and one droplet plate (53). The droplet plate (53) had an angled configuration with an angle of 3°. The droplet plate (53) was made of stainless steel and had a length of 630mm, a width of 128 mm and a thickness of 1mm.

The feed to the spray dryer consisted of 10.45% by weight of acrylic acid, 33.40% by weight of sodium acrylate, 0.018% by weight of 3-tuply ethoxylated glycerol triacrylate, 0.108% by weight of disodium 1-hydroxyethane-1,1-diphosphonic acid (HDPA), 0.072% by weight of [2,2'-azo-bis[2-(2-imidazolin-2-yl)propane]dihydrochloride, 0.072% by weight of sodiumperoxodisulfate solution (15% by weight in water) and water. The degree of neutralization was 71%. The feed per bore was 1.4 kg/h.

The resulting superabsorbent polymer particles were analyzed. The conditions and results are summarized in tables 1 to 3.

Example 2 (not inventive)

1200 g of the water-absorbent polymer particles prepared in Example 1 (base polymer) were put into a laboratory ploughshare mixer (model MR5, manufactured by Gebrüder Lödige Maschinenbau GmbH, Paderborn, Germany). A surface-postcrosslinker solution was prepared by mixing 30 g ethylene carbonate, 1.2 g aluminum sulfate and 60 g of deionized water, as described in Table 4, into a beaker. At a mixer speed of 200 rpm, the aqueous solution was sprayed onto the polymer particles within one minute by means of a spray nozzle. The mixing was continued for additional 5 minutes. The product was removed and transferred into another ploughshare mixer (model MR5, manufactured by Gebrüder Lödige Maschinenbau GmbH; Paderborn; Germany) which was heated to 150° C before. After mixing for further 40 minutes at 150° C, the product was removed from the mixer and sifted through a 850 $\mu$m sieve.

A remoisturizing solution was prepared by mixing 3 g aluminum lactate, 25 mg Sorbitanmonododecanoate (Span® 20) and 80 g of deionized water, as described in Table 8, into a beaker. 1000 g of this product was transferred into another ploughshare mixer (model MR5, manufactured by Gebrüder Lödige Maschinenbau GmbH; Paderborn; Germany) which was heated to 80° C before. After reaching the final temperature of 80° C the heating of the ploughshare mixer was switch off and at a mixer speed of 200 rpm, the aqueous remoisturizing solution was sprayed onto the polymer particles within 2 minutes by means of a spray nozzle. The mixing was continued for additional 13 minutes. The product was removed and sifted through a 850 $\mu$m sieve.

The samples were analyzed. The formulation, conditions and results are summarized in Table 4 and 5.

Example 3 (not inventive)

The example was performed analogous to Example 2, except, that additionally 0.18 g hydrogene peroxide was added into the surface-postcrosslinker solution.

Example 4 (inventive)

The example was performed analogous to Example 2, except, that additionally 0.36 g hydrogene peroxide was added into the surface-postcrosslinker solution.

Example 5 (inventive)

The example was performed analogous to Example 2, except, that additionally 0.72 g hydrogene peroxide was added into the surface-postcrosslinker solution.

Example 6 (not inventive)

The example was performed analogous to Example 2, except, that additionally 1.08 g hydrogene peroxide was added into the surface-postcrosslinker solution.

Example 7 (not inventive)

The example was performed analogous to Example 2, except, that additionally 1.80 g hydrogene peroxide was added into the surface-postcrosslinker solution.

Example 8 (not inventive)

The example was performed analogous to Example 2, except, that additionally 3.60 g hydrogene peroxide was added into the surface-postcrosslinker solution.

Example 9 (not inventive)

The example was performed analogous to Example 2, to Example 2, except, that additionally 0.18 g sodium persulfate was added into the surface-postcrosslinker solution.

Example 10 (not inventive)

The example was performed analogous to Example 2, to Example 2, except, that additionally 0.36 g sodium persulfate peroxide was added into the surface-postcrosslinker solution.

Example 11 (not inventive)

The example was performed analogous to Example 2, to Example 2, except, that additionally 0.72 g sodium persulfate peroxide was added into the surface-postcrosslinker solution.

Example 12 (not inventive)

The example was performed analogous to Example 2, to Example 2, except, that additionally 0.15 g hydrogene peroxide was added into the remoisturizer solution.

Example 13 (not inventive)

The example was performed analogous to Example 2, to Example 2, except, that additionally 0.30 g hydrogene peroxide was added into the remoisturizer solution.

Example 14 (not inventive)

The example was performed analogous to Example 2, to Example 2, except, that additionally 0.60 g hydrogene peroxide was added into the remoisturizer solution.

Tab. 1: Process conditions of the polymerization (base polymer)

|  | T gas inlet | T gas outlet | T gas IFB | T IFB | T CC | T GDU |
|---|---|---|---|---|---|---|
| Unit | ° C | ° C | ° C | ° C | ° C | ° C |
| Example 1 | 167 | 114 | 105 | 71 | 56 | 47 |

T gas inlet:     temperature of the gas prior to the gas distributor (3)
T gas outlet:     temperature of the gas leaving the reaction zone (5)
T gas IFB     temperature of the gas entering the internal fluidized bed (27) via line (25)
T IFB:     temperature of the superabsorbent polymer particles in the fluidized bed (27)
T CC:     temperature of the gas leaving the condenser column (12)
T GDU:     temperature of the gas leaving the gas drying unit (37)

Tab. 2: Properties of the superabsorbent polymer particles (base polymer)

|  | Bulk Density | Flowrate | CRC | AUL | RAA | Ext. 16h | Moisture |
|---|---|---|---|---|---|---|---|
| Unit | $kg/m^3$ | g/s | g/g | g/g | wt.% | wt.% | wt.% |
| Example 1 | 676 | 10.9 | 43.9 | 26.6 | 0.588 | 4.4 | 8.8 |
| *) comparative example | | | | | | | |

Tab. 3: Particle size distribution of the superabsorbent polymer particles (base polymer)

| | 150 | 150-200 | 200-250 | 250-300 | 300-400 | 400-500 | 500-600 | 600-710 | 710-850 | > 850 | Roundness |
|---|---|---|---|---|---|---|---|---|---|---|---|
| Unit | μm | μm | μm | μm | μm | μm | μm | μm | μm | μm | |
| Example 1 | 0.0 | 0.3 | 2.5 | 6.6 | 29.4 | 31.2 | 18.4 | 8.2 | 2.8 | 0.6 | 0.82 |

Tab. 4: Process conditions of the surface post-crosslinking (SXL)

| Example | SXL | | | | | | Cooler / Remoisturizing | | | |
|---|---|---|---|---|---|---|---|---|---|---|
| | Temp. | EC | Water | Al-Sulfate | H$_2$O$_2$ | NaPS | Water | Al-Lactate | Span®20 | H$_2$O$_2$ |
| | ° C | % bop | % bop | % bop | ppm bop | ppm bop | % bop | % bop | ppm bop | ppm bop |
| 2*) | 150 | 2 | 5 | 0,1 | 0 | | 8 | 0,3 | 25 | |
| 3*) | 150 | 2 | 5 | 0,1 | 150 | | 8 | 0,3 | 25 | |
| 4 | 150 | 2 | 5 | 0,1 | 300 | | 8 | 0,3 | 25 | |
| 5 | 150 | 2 | 5 | 0,1 | 600 | | 8 | 0,3 | 25 | |
| 6*) | 150 | 2 | 5 | 0,1 | 900 | | 8 | 0,3 | 25 | |
| 7*) | 150 | 2 | 5 | 0,1 | 1500 | | 8 | 0,3 | 25 | |
| 8*) | 150 | 2 | 5 | 0,1 | 3000 | | 8 | 0,3 | 25 | |
| 9*) | 150 | 2 | 5 | 0,1 | | 150 | 8 | 0,3 | 25 | |
| 10*) | 150 | 2 | 5 | 0,1 | | 300 | 8 | 0,3 | 25 | |
| 11*) | 150 | 2 | 5 | 0,1 | | 600 | 8 | 0,3 | 25 | |
| 12*) | 150 | 2 | 5 | 0,1 | | | 8 | 0,3 | 25 | 150 |
| 13*) | 150 | 2 | 5 | 0,1 | | | 8 | 0,3 | 25 | 300 |
| 14*) | 150 | 2 | 5 | 0,1 | | | 8 | 0,3 | 25 | 600 |

EC:           ethylene carbonate      Span®20:      Sorbitanmonododecanoate

Al-Lactate:      aluminum trilactate      H$_2$O$_2$:      hydrogene peroxide

Al-Sulfate: *)      aluminum sulfate      bop:      based on polymer

                 comparative example

Tab. 5: Properties of the superabsorbent polymer particles (after surface post-crosslinking) and color stability of the superabsorbent polymer particles, stored at 70° C and 80% relative humidity in a climatic test cabinet for 0, 7 and 14 days

| Example | Performance | | | Color (70° C and 80% humidity) | | | | | | | | | | | | |
|---|---|---|---|---|---|---|---|---|---|---|---|---|---|---|---|---|
| | CRC | AUHL | Caking | L | | | a | | | b | | | YI | | |
| | g/g | g/g | % | 0 d | 7 d | 14 d | 0 d | 7 d | 14 d | 0 d | 7 d | 14 d | 0 d | 7 d | 14 d |
| 2*) | 37,0 | 24,5 | 4,4 | 91,2 | 73,3 | 63,0 | -1,5 | 3,6 | 6,4 | 9,7 | 15,4 | 18,0 | 17,7 | 40,8 | 58,2 |
| 3*) | 37,7 | 24,7 | 3,3 | 91,8 | 75,4 | 65,0 | -1,5 | 3,1 | 5,8 | 6,9 | 14,5 | 18,0 | 12,3 | 37,2 | 55,8 |
| 4 | 40,4 | 24,5 | 4,6 | 93,6 | 77,1 | 67,6 | -1,5 | 2,7 | 5,2 | 6,0 | 13,3 | 16,8 | 10,3 | 33,3 | 50,0 |
| 5 | 42,8 | 23,5 | 4,7 | 94,2 | 79,1 | 71,6 | -1,4 | 1,8 | 3,9 | 6,0 | 13,7 | 18,0 | 10,3 | 32,7 | 48,7 |
| 6*) | 47,4 | 18,5 | 5,1 | 92,8 | 76,5 | 66,1 | -1,8 | 3,0 | 5,8 | 6,0 | 13,1 | 17,0 | 10,2 | 33,3 | 52,2 |
| 7*) | 61,9 | 7,7 | 4,8 | 94,5 | 74,6 | 61,9 | -2,1 | 3,1 | 6,2 | 7,2 | 14,2 | 17,4 | 12,0 | 37,0 | 57,2 |
| 8*) | 36,5 | 4,9 | 5,7 | 92,3 | 51,8 | 31,8 | -2,7 | 5,9 | 7,4 | 10,3 | 15,1 | 10,9 | 17,8 | 60,3 | 77,7 |
| 9*) | 38,6 | 23,3 | 4,9 | 90,1 | 75,3 | 66,3 | -1,8 | 2,8 | 5,5 | 12,1 | 15,3 | 17,9 | 22,5 | 38,8 | 54,2 |
| 10*) | 39,8 | 22,2 | 4,9 | 90,4 | 75,3 | 66,1 | -1,8 | 2,8 | 5,5 | 11,0 | 15,2 | 18,1 | 20,5 | 38,6 | 54,9 |
| 11*) | 38,7 | 19,8 | 4,4 | 89,3 | 73,5 | 63,7 | -1,7 | 3,3 | 6,2 | 13,3 | 15,6 | 18,1 | 25,3 | 41,0 | 57,8 |
| 12*) | 37,6 | 25,3 | 5,1 | 92,0 | 73,1 | 63,0 | -1,4 | 3,6 | 6,3 | 9,6 | 14,7 | 17,2 | 17,6 | 39,6 | 55,9 |
| 13*) | 37,4 | 25,8 | 5,2 | 91,0 | 76,0 | 69,7 | 2,0 | 1,8 | 3,3 | 2,6 | 16,7 | 20,7 | 6,7 | 41,0 | 56,5 |

(continued)

| | Performance | | | Color (70° C and 80% humidity) | | | | | | | | | | | |
|---|---|---|---|---|---|---|---|---|---|---|---|---|---|---|---|
| | CRC | AUHL | Caking | L | | | a | | | b | | | YI | | |
| Example | g/g | g/g | % | 0 d | 7 d | 14 d | 0 d | 7 d | 14 d | 0 d | 7 d | 14 d | 0 d | 7 d | 14 d |
| 14*) | 36,8 | 25,4 | 3,1 | 92,9 | 71,1 | 59,1 | -1,4 | 4,2 | 6,6 | 6,0 | 14,6 | 17,1 | 10,6 | 41,0 | 59,6 |
| *) comparative example | | | | | | | | | | | | | | | |

## Claims

1. A process for producing long-term color stable superabsorbent polymer particles, comprising polymerization of a monomer solution, comprising

   a) partly neutralized acrylic acid,
   b) 0.05 to 1.5% by weight, based on acrylic acid prior to neutralization, of at least one crosslinker, and
   c) at least one initiator,

   drying the resulting polymer gel, optionally grinding and classifying the resulting dried polymer gel and thermally post-crosslinking and cooling the resulting polymer particles, wherein 0.005 to 5% by weight of a thermal post-crosslinker and 0.016 to 0.080% by weight of hydrogen peroxide, based in each case on the polymer particles, are added to the polymer particles prior to the thermal post-crosslinking.

2. The process according to claim 1, wherein from 0.02 to 0.05% by weight of the peroxide, based on the polymer particles, is added to the polymer particles prior to the post-crosslinking.

3. The process according to claims 1 or 2, wherein the temperature of the post-crosslinking is from 130 to 190°C.

4. The process according to any of claims 1 to 3, wherein the residence time of the post-crosslinking is from 20 to 60 minutes.

5. The process according to any of claims 1 to 4, wherein the monomer solution comprises a polymerization inhibitor.

6. The process according to any of claims 1 to 5, wherein the monomer solution comprises from 0.004 to 0.010% by weight of a polymerization inhibitor, based on acrylic acid.

7. The process according to claims 5 or 6, wherein hydroquinone monomethyl ether is used as polymerization inhibitor.

8. The process according to any of claims 1 to 7, wherein at least one chelating agent is added to is added to the polymer particles during or after the cooling.

9. The process according to any of claims 1 to 8, wherein from 0.005 to 1% by weight of at least one chelating agent, based on the polymer particles, is added to the polymer particles during or after the cooling.

10. The process according to claims 8 or 9, wherein 1-hydroxyethane-1,1-diphosphonic acid and/or salts thereof are used as chelating agent.

11. The process according to any of claims 1 to 10, wherein the post-crosslinking is without any irradiation with active energy rays.

12. Superabsorbent polymer particles obtainable according to a process of any of claims 1 to 11, wherein the superabsorbent polymer particles have a centrifuge retention capacity of at least 40 g/g, an absorption under high load of at least 20 g/g and a yellowness index after 14 days of aging at 70°C and a relative humidity of 80% of not more than 54, wherein the centrifuge retention capacity of superabsorbent polymer particles is determined by the EDANA recommended test method No. WSP 241.2 (05) "Gravimetric Determination of Fluid Retention Capacity in Saline Solution After Centrifugation", wherein for higher values of the centrifuge retention capacity lager tea bags have to be used, the absorption under high load of the water-absorbent polymer particles is determined analogously to the EDANA recommended test method No. WSP 242.2 (05) "Gravimetric Determination of Absorption Under Pressure", except using a weight of 49.2 $g/cm^2$ instead of a weight of 21.0 $g/cm^2$ and the yellowness index of YI D1925 (2/C) is measured per ASTM D-1925, 2 deg./III. °C.

13. The superabsorbent polymer particles according to claim 12, wherein the superabsorbent polymer particles have a yellowness index after 14 days of aging at 70°C and a relative humidity of 80% of not more than 50, wherein the yellowness index of YI D1925 (2/C) is measured per ASTM D-1925, 2 deg./III.°C.

**Patentansprüche**

1. Verfahren zur Herstellung langzeitfarbstabiler superabsorbierender Polymerpartikel, umfassend die Polymerisation einer Monomerlösung, umfassend

   a) teilweise neutralisierte Acrylsäure,
   b) 0,05 bis 1,5 Gew.-%, bezogen auf Acrylsäure vor der Neutralisation, mindestens eines Vernetzers, und
   c) mindestens einen Initiator,

   Trocknen des resultierenden Polymergels, optionales Mahlen und Klassieren des resultierenden getrockneten Polymergels sowie thermisches Nachvernetzen und Abkühlen der resultierenden Polymerpartikel, wobei 0,005 bis 5 Gew.-% eines thermischen Nachvernetzers und 0,016 bis 0,080 Gew.-% Wasserstoffperoxid, jeweils bezogen auf die Polymerpartikel, den Polymerpartikeln vor dem thermischen Nachvernetzen zugesetzt werden.

2. Verfahren nach Anspruch 1, wobei 0,02 bis 0,05 Gew.-% des Peroxids, bezogen auf die Polymerpartikel, den Polymerpartikeln vor dem Nachvernetzen zugesetzt werden.

3. Verfahren nach den Ansprüchen 1 oder 2, wobei die Temperatur des Nachvernetzungsprozesses 130 bis 190 °C beträgt.

4. Verfahren nach einem der Ansprüche 1 bis 3, wobei die Verweilzeit des Nachvernetzungsprozesses 20 bis 60 Minuten beträgt.

5. Verfahren nach einem der Ansprüche 1 bis 4, wobei die Monomerlösung einen Polymerisationsinhibitor umfasst.

6. Verfahren nach einem der Ansprüche 1 bis 5, wobei die Monomerlösung 0,004 bis 0,010 Gew.-% eines Polymerisationsinhibitors, bezogen auf Acrylsäure, umfasst.

7. Verfahren nach den Ansprüchen 5 oder 6, wobei Hydrochinonmonomethylether als Polymerisationsinhibitor verwendet wird.

8. Verfahren nach einem der Ansprüche 1 bis 7, wobei während oder nach dem Abkühlen mindestens ein Chelatbildner den Polymerpartikeln zugesetzt wird.

9. Verfahren nach einem der Ansprüche 1 bis 8, wobei 0,005 bis 1 Gew.-% mindestens eines Chelatbildners, bezogen auf die Polymerpartikel, den Polymerpartikeln während oder nach dem Abkühlen zugesetzt wird.

10. Verfahren nach den Ansprüchen 8 oder 9, wobei 1-Hydroxyethan-1,1-diphosphonsäure und/oder Salze davon als Chelatbildner verwendet werden.

11. Verfahren nach einem der Ansprüche 1 bis 10, wobei das Nachvernetzen ohne jegliche Bestrahlung mit aktiven Energiestrahlen erfolgt.

12. Superabsorbierende Polymerpartikel, erhältlich nach einem Verfahren nach einem der Ansprüche 1 bis 11, wobei die superabsorbierenden Polymerpartikel eine Zentrifugen-Retentionskapazität von mindestens 40 g/g, eine Absorption unter hoher Last von mindestens 20 g/g und einen Gelblichkeitsindex nach 14 Tagen Alterung bei 70 °C und einer relativen Luftfeuchtigkeit von 80 % von nicht mehr als 54 aufweisen, wobei die Zentrifugen-Retentionskapazität der superabsorbierenden Polymerpartikel gemäß der von EDANA empfohlenen Prüfmethode Nr. WSP 241.2 (05) "Gravimetric Determination of Fluid Retention Capacity in Saline Solution After Centrifugation" bestimmt wird, wobei für höhere Werte der Zentrifugen-Retentionskapazität größere Teebeutel zu verwenden sind, die Absorption unter hoher Last der wasserabsorbierenden Polymerpartikel analog zur von EDANA empfohlenen Prüfmethode Nr. WSP 242.2 (05) "Gravimetric Determination of Absorption Under Pressure" bestimmt wird, mit der Ausnahme, dass ein Gewicht von 49,2 g/cm$^2$ anstelle eines Gewichts von 21,0 g/cm$^2$ verwendet wird, und der Gelblichkeitsindex YI D1925 gemäß ASTM D-1925, 2 deg./III.°C, gemessen wird.

13. Superabsorbierende Polymerpartikel nach Anspruch 12, wobei die superabsorbierenden Polymerpartikel einen Gelblichkeitsindex nach 14 Tagen Alterung bei 70 °C und einer relativen Luftfeuchtigkeit von 80 % von nicht mehr als 50 aufweisen, wobei der Gelblichkeitsindex YI D1925, 2 deg./III.°C, gemäß ASTM D-1925 gemessen wird.

**Revendications**

1. Procédé de production de particules de polymère superabsorbant présentant une stabilité de couleur à long terme, comprenant la polymérisation d'une solution monomère, comprenant

   a) de l'acide acrylique partiellement neutralisé,
   b) de 0,05 à 1,5 % en poids, par rapport à l'acide acrylique avant neutralisation, d'au moins un agent de réticulation, et
   c) au moins un initiateur,

   le séchage du gel polymère obtenu, le broyage et le classement facultatifs du gel polymère séché obtenu, ainsi que la post-réticulation thermique et le refroidissement des particules polymères obtenues, dans lequel 0,005 à 5 % en poids d'un agent de post-réticulation thermique et 0,016 à 0,080 % en poids de peroxyde d'hydrogène, chacun par rapport aux particules polymères, sont ajoutés aux particules polymères avant la post-réticulation thermique.

2. Procédé selon la revendication 1, dans lequel de 0,02 à 0,05 % en poids du peroxyde, par rapport aux particules polymères, est ajouté aux particules polymères avant la post-réticulation.

3. Procédé selon les revendications 1 ou 2, dans lequel la température de la post-réticulation est de 130 à 190 °C.

4. Procédé selon l'une quelconque des revendications 1 à 3, dans lequel le temps de séjour de la post-réticulation est de 20 à 60 minutes.

5. Procédé selon l'une quelconque des revendications 1 à 4, dans lequel la solution monomère comprend un inhibiteur de polymérisation.

6. Procédé selon l'une quelconque des revendications 1 à 5, dans lequel la solution monomère comprend de 0,004 à 0,010 % en poids d'un inhibiteur de polymérisation, par rapport à l'acide acrylique.

7. Procédé selon les revendications 5 ou 6, dans lequel l'éther monométhylique de l'hydroquinone est utilisé comme inhibiteur de polymérisation.

8. Procédé selon l'une quelconque des revendications 1 à 7, dans lequel au moins un agent chélatant est ajouté aux particules polymères pendant ou après le refroidissement.

9. Procédé selon l'une quelconque des revendications 1 à 8, dans lequel de 0,005 à 1 % en poids d'au moins un agent chélatant, par rapport aux particules polymères, est ajouté aux particules polymères pendant ou après le refroidissement.

10. Procédé selon les revendications 8 ou 9, dans lequel l'acide 1-hydroxyéthane-1,1-diphosphonique et/ou des sels de celui-ci sont utilisés comme agent chélatant.

11. Procédé selon l'une quelconque des revendications 1 à 10, dans lequel la post-réticulation est effectuée sans irradiation par des rayonnements d'énergie active.

**12.** Particules de polymère superabsorbant obtenables selon un procédé de l'une quelconque des revendications 1 à 11, dans lequel les particules de polymère superabsorbant présentent une capacité de rétention par centrifugation d'au moins 40 g/g, une absorption sous charge élevée d'au moins 20 g/g et un indice de jaunissement, après 14 jours de vieillissement à 70 °C et une humidité relative de 80 %, n'excédant pas 54, dans lequel la capacité de rétention par centrifugation des particules de polymère superabsorbant est déterminée selon la méthode d'essai recommandée par l'EDANA n° WSP 241.2 (05) "Gravimetric Determination of Fluid Retention Capacity in Saline Solution After Centrifugation", dans laquelle, pour des valeurs plus élevées de la capacité de rétention par centrifugation, des sachets de thé de plus grande taille doivent être utilisés, l'absorption sous charge élevée des particules polymères absorbantes d'eau étant déterminée de manière analogue à la méthode d'essai recommandée par l'EDANA n° WSP 242.2 (05) "Gravimetric Determination of Absorption Under Pressure", à l'exception de l'utilisation d'un poids de 49,2 g/cm$^2$ au lieu d'un poids de 21,0 g/cm$^2$, et l'indice de jaunissement YI D1925, 2 deg./III.°C, étant mesuré conformément à l'ASTM D-1925.

**13.** Particules de polymère superabsorbant selon la revendication 12, dans lequel les particules de polymère superabsorbant présentent un indice de jaunissement, après 14 jours de vieillissement à 70 °C et une humidité relative de 80 %, n'excédant pas 50, dans lequel l'indice de jaunissement YI D1925, 2 deg./III.°C, est mesuré conformément à l'ASTM D-1925.

**EP 3 873 974 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1770113 A1
- WO 2006062258 A2
- WO 2010096595 A2
- EP 2915548 A1
- CN 101045789 A
- CN 102311599 A
- WO 2016134905 A1
- CN 104744711 A
- US 2007078231 A1
- EP 0248437 A2
- US 2010298513 A1
- EP 0530438 A1
- EP 0547847 A1
- EP 0559476 A1
- EP 0632068 A1
- WO 9321237 A1
- WO 2003104299 A1
- WO 2003104300 A1
- WO 2003104301 A1
- DE 10331450 A1
- DE 10331456 A1
- DE 10355401 A1
- DE 19543368 A1
- DE 19646484 A1
- WO 9015830 A1
- WO 2002032962 A2
- WO 2001038402 A1
- DE 3825366 A1
- US 6241928 B
- WO 2008052971 A1
- WO 2011026876 A1
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 2003031482 A1
- DE 3713601 A1

**Non-patent literature cited in the description**

- **F.L. BUCHHOLZ** ; **A.T. GRAHAM**. Modern Super-absorbent Polymer Technology. Wiley-VCH, 1998, 71-103
- *Hunterlab*, 1996, vol. 8 (7), 1-4